(19) Europäisches Patentamt
European Patent Office
Office européen des brevets

(11) Veröffentlichungsnummer: **0 464 472 A2**

(12) **EUROPÄISCHE PATENTANMELDUNG**

(21) Anmeldenummer: **91110056.8**

(22) Anmeldetag: **19.06.91**

(51) Int. Cl.5: **C07C 22/08**, C07C 39/367, C07C 37/16, C07C 17/26

(30) Priorität: **25.06.90 DE 4020184**

(43) Veröffentlichungstag der Anmeldung: **08.01.92 Patentblatt 92/02**

(84) Benannte Vertragsstaaten: **CH DE FR GB IT LI**

(71) Anmelder: **HOECHST AKTIENGESELLSCHAFT**
**Postfach 80 03 20**
**W-6230 Frankfurt am Main 80(DE)**

(72) Erfinder: **Appel, Wolfgang**
**Taunusstrasse 74A**
**W-6233 Kelkheim (Taunus)(DE)**
Erfinder: **Siegemund, Günter**
**Frankfurter Strasse 21**
**W-6238 Hofheim(DE)**

# (54) Teilfluorierte Biphenyle, Verfahren zu ihrer Herstellung und ihre Verwendung.

(57) Verbindungen der Formel

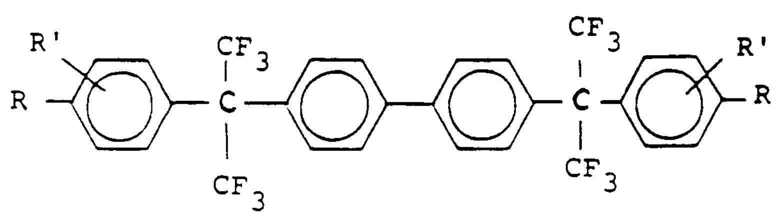

(I),

in der unabhängig voneinander R gleich OH oder niederes Alkyl mit 1 - 4 C-Atomen und R' gleich Wasserstoff oder niederes Alkyl mit 1 - 4 C-Atomen ist, werden hergestellt durch Kondensation von teilfluorierten aromatischen Kohlenwasserstoffen in Gegenwart von Fluorwasserstoff oder durch reduktive Kupplung von teilfluorierten Aromaten. Die Verbindungen werden als Ausgangsmaterial zur Herstellung von teilfluorierten Polykondensaten verwendet.

EP 0 464 472 A2

Die Erfindung betrifft neue, teilfluorierte Biphenyle, Verfahren zu ihrer Herstellung und ihre Verwendung als Zwischenprodukt zur Synthese von teilfluorierten Monomeren und teilfluorierten Polykondensaten.

Biphenyle, die 2 Hexafluorisopropylgruppen mit Aminophenylresten enthalten, sind als Härterkomponente von Epoxidharzen für Verbundwerkstoffe und als Komponente von Polyimidverbundwerkstoffen bekannt (EP-B-0 126 494 und JP-A-63,199,237).

Weiterhin wird ein Verfahren zur Darstellung von Polyketonen in Gegenwart von Fluoralkansulfonsäuren beschrieben, wobei als eine der Reaktionskomponenten aromatische Disäurehalogenide eingesetzt werden, die, gemäß einer allgemeinen Formel mit mehreren Variablen, auch auf teilfluorierten Biphenylen basieren können (EP-A-0 063 874). Für den Acylierungsprozeß sind dabei Säurehalogenide unbedingt erforderlich, die entsprechenden freien Säuren können daher nicht verwendet werden.

Ferner sind teilfluorierte Diphenylether bekannt, die in Gegenwart von Fluorwasserstoff hergestellt werden und als Komponente zur Synthese von Monomeren und Polykondensaten Verwendung finden (DE-A-37 39 795).

Gegenstand der Erfindung sind Verbindungen der Formel

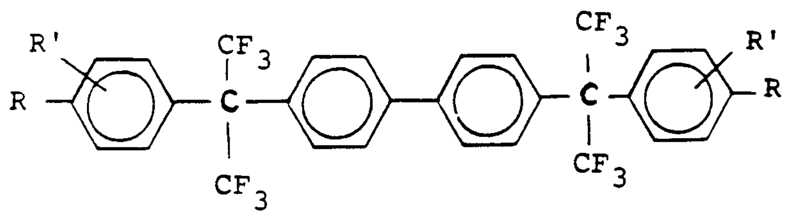

(I),

in der unabhängig voneinander R gleich OH oder niederes Alkyl mit 1 - 4 C-Atomen und R' gleich Wasserstoff oder niederes Alkyl mit 1 - 4 C-Atomen ist, wobei Alkyl vorzugsweise $CH_3$ ist, Verfahren zu ihrer Herstellung und ihre Verwendung. In Formel (I) befindet sich der Rest R' vorzugsweise in ortho-Stellung zum Rest R.

Die Verbindungen gemäß der Erfindung können im allgemeinen nach drei verschiedenen Methoden hergestellt werden, und zwar:

a) durch Kondensation von einem Mol eines Dicarbinols der Formel

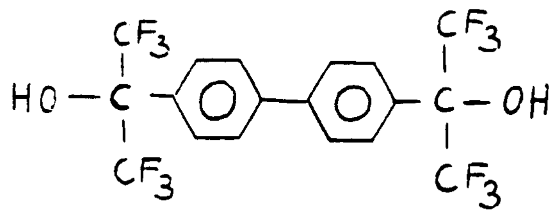

(II)

mit mindestens 2 Mol einer Verbindung mit der Formel

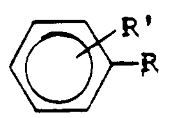

(III),

wobei R die vorgenannte Bedeutung hat,
oder

b) durch Kondensation von mindestens 2 Mol einer Verbindung der Formel

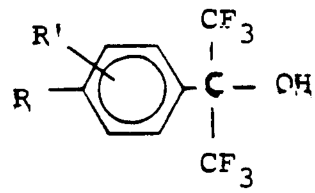

(IV),

wobei R die vorgenannte Bedeutung hat,
mit einem Mol Biphenyl (V), jeweils in Gegenwart von Fluorwasserstoff
oder
c) durch Bildung der Kohlenstoff-Kohlenstoff-Bindung zwischen 2 gleichen teilfluorierten aromatischen Verbindungen der Formel

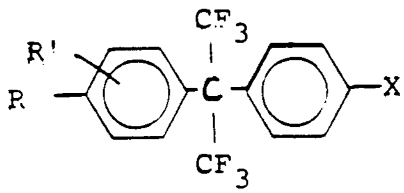

(VI),

wobei R die vorgenannte Bedeutung hat,
nach einem literaturbekannten Verfahren, das sich zur Bildung von Aryl-Arylbindungen eignet, z.B. J. Org. Chem. 51, 2627 (1986). X ist Halogen, vorzugsweise Chlor. Bei Anwendung dieser Synthesemethode ist im speziellen Fall, wenn R gleich OH ist, vor Bildung der Aryl-Arylbindung der Schutz der Hydroxylgruppe notwendig, beispielsweise durch eine Acetylgruppe.

Verbindungen der Formel (II), die beim Vorgehen nach a) eingesetzt werden, sind in der US-Patentschrift 3,355,500 und in J. Org. Chem. 30, 998 (1965) beschrieben. Verbindungen der Formel (IV), die nach Methode b) zu den erfindungsgemäßen Verbindungen umgesetzt werden können, sind ebenfalls in J. Org. Chem. 30, 998-1001 (1965) beschrieben.

Beispiele für aromatische substituierte Kohlenwasserstoffe der Formel (III) sind Phenylverbindungen, die mit OH und/oder Alkylresten mit 1 - 4 C-Atomen substituiert sind, wie Phenol, Toluol, die verschiedenen Xylole und Kresole.

Die Reaktion nach Methode a) und b) wird bei einer Temperatur von 80 bis 180° C, vorzugsweise von 100 bis 160° C, durchgeführt.

Für die Reaktion nach Methode a) und b) ist ein Zeitraum von 20 bis 90 Stunden, vorzugsweise von 40 bis 70 Stunden, erforderlich.

Das Molverhältnis der eingesetzten Reaktionspartner wird bestimmt bei Methode a) durch das Verhältnis der Verbindung (II) zu Verbindung (III), sowie bei Methode b) durch das Verhältnis des Biphenyls zu Verbindung (IV); es beträgt jeweils mindestens 1:2, vorzugsweise 1:2,2 bis 1:4,4.

Der Anteil an Fluorwasserstoff, der bei der Reaktion zur Herstellung der Verbindungen gemäß der Erfindung notwendig ist, wird bei Methode a) auf die Verbindung (II) bezogen und liegt im allgemeinen bei einem Molverhältnis von 1:7 bis 1:25, vorzugsweise 1:8 bis 1:12. Bei der Methode b) beträgt das Molverhältnis der Verbindung (IV) zu Fluorwasserstoff im allgemeinen 1:6 bis 1:15, vorzugsweise 1:8 bis 1:12.

Zur Aufarbeitung des Reaktionsgutes wird im allgemeinen der Fluorwasserstoff nach Beendigung der Reaktion bei ca. 80° C aus dem Reaktor abgegast und der verbleibende Rückstand, gegebenenfalls nach Verdünnen mit einem organischen Lösungsmittel, dem Reaktor entnommen, vorzugsweise bei einer Temperatur von 20-30° C.

Als Lösungsmittel, die dabei verwendet werden können, eignen sich aliphatische Kohlenwasserstoffe mit 5 bis 10 C-Atomen, aromatische Kohlenwasserstoffe mit 6 bis 8 C-Atomen und ein- oder mehrfach chlorierte aliphatische Kohlenwasserstoffe mit 1 bis 4 C-Atomen im Alkylrest. Beispiele hierfür sind n-Hexan, n-Heptan, Toluol, die verschiedenen Xylole, sowie Di- und Trichlormethan, vorzugsweise Toluol, Di- oder Trichlormethan.

Das erhaltene Rohgemisch wird mit Wasser versetzt, gewaschen und abgetrennt. Im allgemeinen fallen die gereinigten Produkte als farblose Kristallisate an.

Zur weiteren Reinigung kann das Reaktionsgut einer Umkristallisation aus einem organischen Lösungsmittel unterworfen werden, oder es erfolgt ein Ausrühren in organischen Lösungsmitteln, vorzugsweise in Isopropanol, Methanol oder 1-Chlorpropan.

Die Darstellung der in Formel (VI) gezeigten Verbindungen ist nach bekannten Verfahren möglich aus Verbindungen der Formel (IV) und Arylhalogeniden.

Die Bildung der Aryl-Arylbindung zwischen zwei Komponenten nach Formel (VI) wird durchgeführt in einem polaren aprotischen Lösungsmittel wie Dimethylacetamid oder Dimethylformamid in Gegenwart eines Gemisches aus 1 - 10 Mol%, vorzugsweise 3 bis 6 Mol% eines Nickel-(II)-Salzes, vorzugsweise $NiCl_2$ oder $NiBr_2$, und 5 - 40 Mol%, vorzugsweise 20 bis 30 Mol%, einer organischen Phosphor-(III)-Verbindung, vorzugsweise Triphenylphosphin, und Zinkpulver in einer Menge von 120 - 160 Mol%, bezogen auf das eingesetzte Arylhalogenid.

Die Reaktion wird in einer Inertgasatmosphäre, insbesondere von Stickstoff oder Argon, bei einer Temperatur von 40 bis 80°C ausgeführt; die Reaktion dauert 2 bis 8 Stunden.

Der Feststoffanteil wird abfiltriert und das Filtrat nach Zusatz eines nicht mit Wasser mischbaren Lösungsmittels, z.B. ein ein- oder mehrfach chlorierter aliphatischer Kohlenwasserstoff mit 1 - 4 Kohlenstoffatomen im Alkylrest, insbesondere Di- oder Trichlormethan, Essigsäureester oder Diethylether, mehrfach mit Wasser gewaschen. Dabei stellt sich eine Phasentrennung ein. Nach Trocknung der organischen Phase wird das Lösungsmittel abdestilliert und das zurückbleibende Produkt durch Umkristallisation gereinigt.

Spezielle neue teilfluorierte Biphenylderivate, die die Erfindung beinhaltet, sind:

4,4'-Bis-[2-(4-hydroxyphenyl)-hexafluorisopropyl]-biphenyl
4,4'-Bis-[2-(4-methylphenyl)-hexafluorisopropyl]-biphenyl
4,4'-Bis-[2-(3,4-dimethylphenyl)-hexafluorisopropyl]-biphenyl

Die neuen Verbindungen dienen vor allem zur Herstellung von Monomeren, die als Komponente teilfluorierter Polykondensate, z.B. von Polyestern, Polyamiden und Polyimiden verwendet werden, wie sie in den Patentanmeldungen vom selben Tage , "Teilfluorierte Tetracarbonsäure sowie deren Dianhydrid, Verfahren zu ihrer Herstellung und ihre Verwendung", DE-P 40 20 186.4, "Teilfluorierte Dicarbonsäure und deren Säurechlorid, Verfahren zu ihrer Herstellung und ihre Verwendung" DE-P 40 20 185.6 beschrieben worden sind.

**Beispiele**

1) <u>4,4'-Bis-[2-(4-methylphenyl)-hexafluorisopropyl]-biphenyl</u>

Synthese nach Methode a)

Ein Zwei-Liter-VA-Rührautoklav (VA = Chrom-Nickel-Stahl) wurde mit 302 g 4,4'-Bis-[hexafluor-2-hydroxy-2-propyl]-biphenyl, 140 g Toluol und 300 g wasserfreiem Fluorwasserstoff gefüllt und 64 Stunden bei 140°C gerührt. Nach Abkühlen auf 80°C wurde der Fluorwasserstoff abgegast. Das Produkt wurde nach Zusatz von 200 ml Toluol aus dem Autoklaven entnommen, die organische Phase mehrfach mit Wasser gewaschen und über $CaCl_2$ getrocknet. Nach Abdestillieren des Lösungsmittels wurde der feste Rückstand aus Methanol umkristallisiert. Die Ausbeute betrug 212 g (54 %) Produkt, Schmelzpunkt 167 - 168°C.

Synthese nach Methode b)

Ein Zwei-Liter-VA-Rührautoklav wurde mit 620 g 2-(4-Methylphenyl)-hexafluorpropanol-2, 154 g Biphenyl und 720 g wasserfreiem Fluorwasserstoff gefüllt und 64 Stunden bei 160°C gerührt. Nach Absenken der Temperatur wurde der Fluorwasserstoff bei 80°C abgegast und das Produkt nach Zusatz von 800 ml Toluol aus dem Autoklaven entnommen. Die organische Phase wurde mehrfach mit Wasser gewaschen und anschließend über $CaCl_2$ getrocknet. Nach Filtration und Entfernung des Lösungsmittels wurden durch Umkristallisieren des Rückstandes aus 2000 ml Methanol 478 g (75 %) Produkt vom Schmelzpunkt 168 - 169°C erhalten.

| Analyse für $C_{32}H_{22}F_{12}$ | | | |
|---|---|---|---|
| | C % | H % | F % |
| ber.: | 60,57 | 3,49 | 35,93 |
| gef.: | 60,60 | 3,40 | 35,60 |

Synthese nach Methode c)

In 150 ml trockenem, entgastem Dimethylacetamid unter einer Stickstoffatmosphäre wurde 2,2 g Nickel-(II)bromid, 15 g Triphenylphosphin und 20 g Zinkstaub vorgelegt und 30 Minuten bei 40°C gerührt. Zu dieser Lösung wurden 70 g 2- (4-Chlorphenyl)-2-(4-methylphenyl)-hexafluorpropan in 150 ml trockenem, entgastem Dimethylacetamid zugesetzt und die Mischung 3 Stunden bei 40 - 50°C gerührt. Bei 20-30°C wurde der Feststoffanteil abfiltriert und mit 300 ml Essigester gewaschen, das Filtrat wurde mehrfach mit Wasser gewaschen und die organische Phase über Magnesiumsulfat getrocknet. Der nach Abdestillieren des Lösungsmittels erhaltene Rückstand wurde aus Methanol umkristallisiert. Die Ausbeute betrug 64 g (50,8 %) an 4,4'-Bis-[2-(4-methylphenyl)-hexafluorisopropyl]-biphenyl vom Schmelzpunkt 168 - 169°C.

2) 4,4'-Bis-[2-(4-hydroxyphenyl)-hexafluorisopropyl]-biphenyl

Synthese nach Methode a)

Ein Zwei-Liter-VA-Rührautoklav wurde mit 302 g 4,4'-Bis-[hexafluor-2-hydroxy-2-propyl]-biphenyl, 140 g Phenol und 300 g wasserfreiem Fluorwasserstoff gefüllt und 48 Stunden bei 110°C gerührt. Nach Abkühlen auf 80°C wurde der Fluorwasserstoff abkondensiert. Das Produkt wurde nach Zusatz von Methylenchlorid aus dem Autoklaven entnommen, die organische Phase mehrfach mit Wasser gewaschen und über $CaCl_2$ getrocknet. Nach Abdestillieren des Lösungsmittels wurde der feste Rückstand durch Umkristallisieren aus Methylenchlorid/n-Hexan (4:1) gereinigt. Die Ausbeute betrug 221 g (55 % d.Th.) Feststoff, Schmelzpunkt 196 - 198°C.

3) 4,4'-Bis-[2-(3,4-dimethylphenyl)-hexafluorisopropyl]-biphenyl

Synthese nach Methode b)

Ein Zwei-Liter-VA-Rührautoklav wurde mit 598 g 2-(3,4-Dimethylphenyl)-hexafluorpropanol-2, 154 g Biphenyl und 720 g wasserfreiem Fuorwasserstoff gefüllt und 70 Stunden bei 150°C gerührt. Nach Absenken der Temperatur auf 80°C wurde der Fluorwasserstoff abgegast und das Produkt nach Zusatz von 500 ml Toluol dem Autoklaven entnommen. Die organische Phase wurde mehrfach mit Wasser gewaschen, über $CaCl_2$ getrocknet und anschließend das Lösungsmittel abdestilliert. Der Rückstand wurde aus 1000 ml Isopropanol unter Zusatz von 20 g Aktivkohle umkristallisiert. Es wurden 385 g Produkt (58 %) vom Schmelzpunkt 164 - 165°C erhalten.

**Patentansprüche**

**1.** Verbindungen der Formel

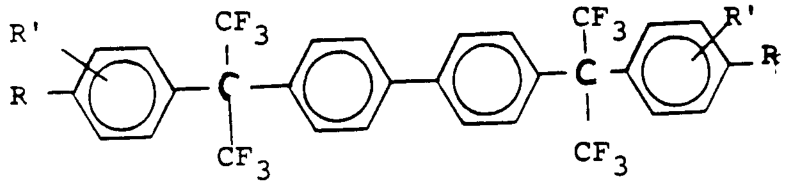

(I)

in der unabhängig voneinander R gleich OH oder niederes Alkyl mit 1 bis 4 C-Atomen, R' gleich Wasserstoff oder niederes Alkyl mit 1 bis 4 C-Atomen ist.

**2.** 4,4'-Bis-[2-(4-hydroxyphenyl)-hexafluorisopropyl]-biphenyl.

**3.** 4,4'-Bis-[2-(4-methylphenyl)-hexafluorisopropyl]-biphenyl.

**4.** 4,4'-Bis-[2-(3,4-dimethylphenyl)-hexafluorisopropyl]-biphenyl.

**5.** Verfahren zur Herstellung einer Verbindung der Formel

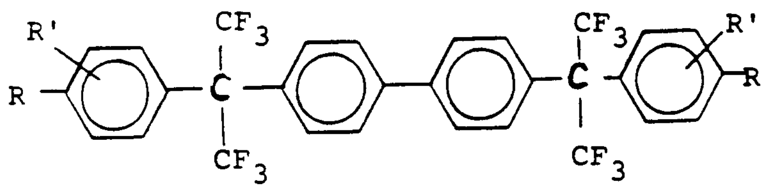

(I)

dadurch gekennzeichnet, daß
a) ein Dicarbinol der Formel

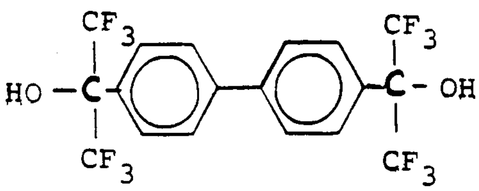

(II)

mit einem aromatischen Kohlenwasserstoff der Formel

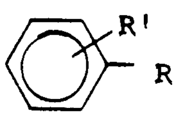

(III)

oder
b) eine Fluorverbindung der Formel

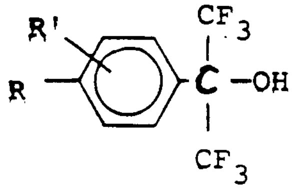

(IV)

mit Biphenyl (V)
- in den Formeln haben R, R' die in Formel (I) genannte Bedeutung -,
in Gegenwart von Fluorwasserstoff bei Temperaturen von 80 bis 180 °C zur Reaktion gebracht werden.

**6.** Verfahren zur Herstellung einer Verbindung der Formel (I), dadurch gekennzeichnet, daß ein Arylhalogenid der Formel

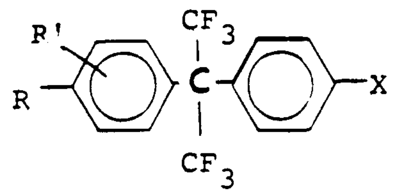

(VI)

- wobei X gleich Chlor oder Brom ist und R und R' die in Formel (I) genannte Bedeutung haben - in Gegenwart eines Gemisches bestehend aus einem Phosphin, metallischem Zink und einem Nickelsalz, mit sich selbst zur Reaktion gebracht wird.

**7.** Ausführungsform nach Anspruch 1, 5 oder 6, dadurch gekennzeichnet, daß das niedere Alkyl die Gruppe $-CH_3$ ist.

**8.** Ausführungsform nach Anspruch 1, 5, 6 oder 7, dadurch gekennzeichnet, daß R' in ortho-Stellung zum Rest R steht.

**9.** Verfahren nach einem oder mehreren der Ansprüche 5, 7 und 8, dadurch gekennzeichnet, daß die Reaktionspartner mindestens im Verhältnis 1:2, vorzugsweise 1:2,2 bis 1:4,4 beim Verfahren a) bezogen auf das Verhältnis von (V) zu (IV), eingesetzt werden.

**10.** Verfahren nach einem oder mehreren der Ansprüche 5, 7 bis 9, dadurch gekennzeichnet, daß das Molverhältnis von Fluorwasserstoff zu Verbindung (II) 7:1 bis 25:1, vorzugsweise 10:1 bis 20:1, insbesondere 8:1 bis 12:1 beträgt.

**11.** Verfahren nach einem oder mehreren der Ansprüche 5, 7 bis 10, dadurch gekennzeichnet, daß die Reaktion bei 100 bis 160 °C durchgeführt wird.

**12.** Verfahren nach einem oder mehreren der Ansprüche 5, 7 bis 11, dadurch gekennzeichnet, daß die Reaktion in 20 bis 90 Stunden, vorzugsweise in 40 bis 70 Stunden, durchgeführt wird.

**13.** Verfahren nach einem oder mehreren der Ansprüche 6 bis 8, dadurch gekennzeichnet, daß der Anteil des Nickel-(II)-Salzes 1 - 10 Mol%, vorzugsweise 3 bis 6 Mol%, des Phosphins 5 - 40 Mol%, vorzugsweise 20 bis 30 Mol% und des Zinks 120 - 160 Mol%, bezogen auf das eingesetzte Arylhalogenid, beträgt.

**14.** Verfahren nach einem oder mehreren der Ansprüche 6 bis 8 und 13, dadurch gekennzeichnet, daß die Reaktion bei einer Temperatur von 40 bis 80 °C durchgeführt wird.

**15.** Verfahren nach einem oder mehreren der Ansprüche 6 bis 8, 13 und 14, dadurch gekennzeichnet, daß die Reaktion 2 bis 8 Stunden dauert.

**16.** Verwendung der Verbindung gemäß Anspruch 1 zur Herstellung von Monomeren, die als Komponente für teilfluorierte Polykondensate, insbesondere teilfluorierte Polyester, Polyamide und Polyimide eingesetzt werden.